# EUROPEAN PATENT SPECIFICATION

(11) **EP 4 142 837 B1**
(45) Date of publication and mention of the grant of the patent: **27.03.2024**
(21) Application number: 21725922.5
(22) Date of filing: 26.04.2021
(51) Int. Cl.: A61M 15/00, B65D 41/00, B65D 55/16, B65D 47/14

(54) **COVER ASSEMBLY AND ACTUATOR FOR INHALER**
ABDECKUNGSANORDNUNG UND AKTUATOR FÜR INHALATOR
ENSEMBLE COUVERCLE ET ACTIONNEUR POUR INHALATEUR

(30) Priority: 30.04.2020 US 202063017707 P
(43) Date of publication of application: 08.03.2023
(73) Proprietor: Kindeva Drug Delivery L.P., Woodbury, MN 55129 (US); Hodson, Peter D., St. Paul, Minnesota 55170 (US); Stuart, Adam J., St. Paul, Minnesota 55170 (US)
(72) Inventor: HODSON, Peter D., St. Paul, Minnesota 55170 (US); STUART, Adam J., St. Paul, Minnesota 55170 (US)
(74) Representative: Vossius & Partner Patentanwälte Rechtsanwälte mbB
(86) International application number: PCT/US2021/029125
(87) International publication number: WO 2021/222087

(56) References cited:
- FR-A1- 2 872 489
- GB-A- 2 364 320
- GB-A- 2 568 982
- US-A- 4 690 332
- US-A1- 2016 101 259
- US-B1- 6 257 231

## Description

### Cross-Reference to Related Application

This application claims the benefit of U.S. Provisional Application No. 63/017,707, filed April 30, 2020.

### Technical Field

The present disclosure relates generally to inhalers and more particularly to cover assemblies and actuators for inhalers.

### Background

Pressurised metered dose inhalers (pMDls) are generally provided with mouthpiece covers in order to protect their internal passageways from contaminants, such as fluff, lint, dust, etc. Conventional press-and-breathe variants of the pMDls are typically provided with unattached mouthpiece covers. The unattached mouthpiece covers may be easily lost (for example, pulled off from the inhaler during transportation or misplaced during use). Moreover, the unattached mouthpiece covers may be swallowed by users who forget to remove them before use, and thereby pose a choking hazard. The unattached mouthpiece covers may also be needed to be put somewhere during use of the inhaler, which may not be convenient during certain circumstances.

In response to the aforementioned problem, current inhalers are produced with captive mouthpiece covers. However, strap-like tethers of the current inhalers may restrict movement of the captive mouthpiece covers. Consequently, users may have limited choice on where the captive mouthpiece cover is positioned during the use of the inhaler. The captive mouthpiece cover may obstruct users' hand(s) or face during use of the inhaler. Moreover, the captive mouthpiece covers do not provide flexibility regarding factors, such as handedness, hand-span, hand strength, ergonomic preferences, face shape, etc.

US 6 257 231 B1 discloses an apparatus comprising a cylindrical aerosol chamber having an input opening and a circular output opening; and an aperture barrier means positioned in the chamber and having circular openings at different sizes.

US 2016/101259 A1 discloses an adapter housing comprising an inlet port, an outlet port, a medicament delivery port, a collar removably disposed around said medicament delivery port and adapted to secure a medicament delivery device to said medicament delivery port, and a valve moveable between a closed position wherein said valve closes said medicament delivery port and an open position wherein said medicament delivery port is open.

US 4 690 332 A discloses an inhaler comprising: an inlet having a first axis for accepting a discharge tube of a container containing a propellant and a medicant; a transducer body having a second central axis along a length of the body and coupled to the inlet wherein the second central axis is normal to a plane containing the first axis and wherein the transducer body comprises an outlet; a central cylindrical bar coaxial with the second central axis, extending substantially the length of the body and having a free end with a groove formed longitudinally in an outer surface of the bar; a lens mounted on the free end of the bar to form a substantially airtight fit between the lens and the outer surface of the bar and at least partly within the body; a first expansion chamber defined by first chamber walls adjacent the body downstream from and adjacent the outlet; a second expansion chamber defined by second chamber walls downstream from and adjacent the first expansion chamber; and a screen mounted to an end of the second expansion chamber.

### Summary

The invention is defined in the appended independent claim. Preferred embodiments are matter of the dependent claims.

In a first aspect, there is provided an actuator for an inhaler. The actuator includes a sleeve having a first end and a second end. The sleeve is substantially hollow and defines a longitudinal axis along its length. The actuator further includes a base formed at the first end of the sleeve. The actuator further includes a mouthpiece joined to the sleeve and the base. The mouthpiece is substantially hollow and defines a delivery opening. The actuator further includes a cover removably attached to the mouthpiece for covering the delivery opening. The actuator further includes a retaining member for retaining the cover to the sleeve upon removal of the cover from the mouthpiece. The retaining member includes a ring disposed around the sleeve. The ring is at least selectively rotatable about the longitudinal axis and slidable along the longitudinal axis relative to the sleeve. The retaining member further includes a strap extending from the ring and attached to the cover.

In some embodiments, the ring is substantially parallel to the longitudinal axis. The ring is both rotatable and slidable relative to the sleeve.

In some embodiments, the ring is movable between a locked position and an unlocked position. In the locked position, the ring is inclined relative to the longitudinal axis and locked with the sleeve to prevent relative movement between the sleeve and the ring. In the unlocked position, the ring is substantially parallel to the longitudinal axis and is both rotatable and slidable relative to the sleeve.

In some embodiments, in the locked position, the ring is inclined towards the base.

In some embodiments, the mouthpiece is inclined relative to the longitudinal axis.

In some embodiments, the mouthpiece is substantially perpendicular to the longitudinal axis.

In some embodiments, the sleeve further includes a protrusion at the second end thereof.

The protrusion is inclined relative to the longitudinal axis.

In some embodiments, the strap is deformable.

In some embodiments, a diameter of the ring is greater than a width of the sleeve.

In a second aspect, there is provided an inhaler. The inhaler includes an actuator. The actuator includes a sleeve having a first end and a second end. The sleeve is substantially hollow and defines a longitudinal axis along its length. The actuator further includes a base formed at the first end of the sleeve. The actuator further includes a mouthpiece joined to the sleeve and the base. The mouthpiece is substantially hollow and defines a delivery opening. The actuator further includes a cover removably attached to the mouthpiece for covering the delivery opening. The actuator further includes a retaining member for retaining the cover to the sleeve upon removal of the cover from the mouthpiece. The retaining member includes a ring disposed around the sleeve. The ring is at least selectively rotatable about the longitudinal axis and slidable along the longitudinal axis relative to the sleeve. The retaining member further includes a strap extending from the ring and attached to the cover. The inhaler further includes a canister containing a fluid formulated with a medicament. The canister is at least partially received within the sleeve.

In a third aspect, there is disclosed, but not claimed, a method of using the inhaler according to the second aspect. The method includes detaching the cover from the mouthpiece to at least partially expose the delivery opening. The method further includes rotating the ring relative to the sleeve to move the cover away from the mouthpiece. The method further includes actuating the inhaler to deliver the medicament through the delivery opening.

In some aspects, the method further includes sliding the ring towards the second end of the sleeve.

In a fourth aspect, there is disclosed a cover assembly for an actuator of an inhaler. The actuator includes a sleeve, a base and a mouthpiece. The cover assembly includes a cover removably attached to the mouthpiece for covering a delivery opening of the mouthpiece. The cover assembly further includes a retaining member for retaining the cover to the sleeve upon removal of the cover from the mouthpiece. The retaining member includes a ring disposed around the sleeve. The ring is at least selectively rotatable about a longitudinal axis of the sleeve and slidable along the longitudinal axis relative to the sleeve. The retaining member further includes a strap extending from the ring and attached to the cover.

### Brief Description of the Drawings

Exemplary embodiments disclosed herein may be more completely understood in consideration of the following detailed description in connection with the following figures. The figures are not necessarily drawn to scale. Like numbers used in the figures refer to like components. However, it will be understood that the use of a number to refer to a component in a given figure is not intended to limit the component in another figure labelled with the same number.
**Figure 1** is a schematic sectional side view of an inhaler including an actuator according to one embodiment of the present disclosure;
**Figure 2** is a schematic side view of the actuator according to one embodiment of the present disclosure;
**Figure 3A** is a schematic side view of the actuator with a cover of a mouthpiece at a first position according to one embodiment of the present disclosure;
**Figure 3B** is a schematic side view of the actuator with a cover of a mouthpiece at a second position according to one embodiment of the present disclosure;
**Figure 4A** is a schematic side view of the actuator with a ring in a locked position according to one embodiment of the present disclosure; and
**Figure 4B** is a schematic side view of the actuator with the ring in an unlocked position according to one embodiment of the present disclosure.

### Detailed Description

In the following description, reference is made to the accompanying figures that form a part thereof and in which various embodiments are shown by way of illustration. It is to be understood that other embodiments are contemplated and may be made without departing from the scope or spirit of the present disclosure. The following detailed description, therefore, is not to be taken in a limiting sense.

The present invention will be described with respect to particular embodiments and with reference to certain drawings, but the invention is not limited thereto. The drawings described are only schematic and are non-limiting. In the drawings, the size of some of the elements may for illustrative purposes be exaggerated and not drawn to scale.

It will be understood that the terms "vertical", "horizontal", "top", "bottom", "above", "below", "left", "right" etc. as used herein refer to particular orientations of the figures and these terms are not limitations to the specific embodiments described herein.

Typically, pressurised metered dose inhaler (pMDI) actuators include a sleeve in which a canister containing a fluid formulated with a medicament is disposed. The pMDI actuator may also include a mouthpiece angled with respect to a longitudinal axis defined along a length of the sleeve. Furthermore, at a base of the sleeve, there may be a nozzle block that includes a stem socket and an exit orifice or actuator nozzle. At the bottom of the actuator, a thumb grip may be provided. The mouthpiece may have a circular, elliptical or oblong cross-section.

During normal operation of an inhaler (an actuator and a canister containing medicament), a plume of medicament is produced from the exit orifice or the actuator nozzle into the mouthpiece and is inhaled by a user through a delivery opening defined by the mouthpiece. The mouthpiece may be covered by a cover removably attached to the mouthpiece, which is detached during the use of the inhaler.

The present disclosure provides a retaining member for retaining the cover to the sleeve upon removal of the cover from the mouthpiece. The retaining member may allow movement of the cover away from the mouthpiece such that the cover may not interfere with the normal operation of the inhaler.

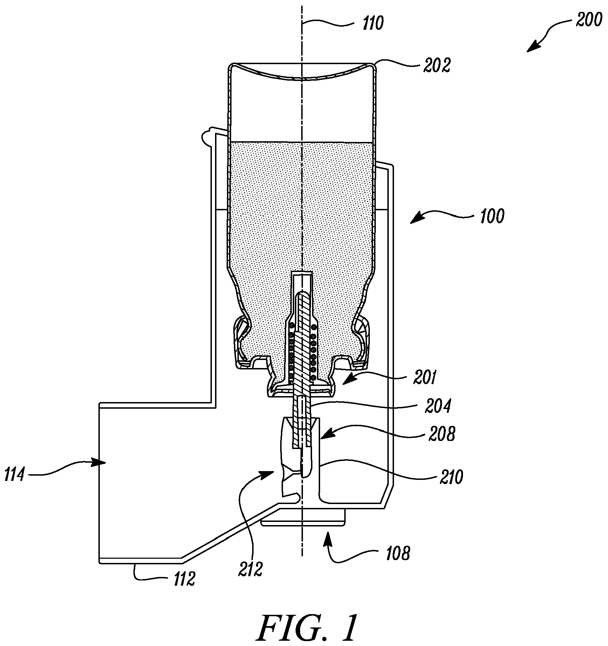

Referring to Figure 1, an inhaler 200 includes an actuator 100. The inhaler 200 also includes a canister 202 containing a fluid formulated with a medicament. The canister 202 may have a generally cylindrical structure. Further, the canister 202 may have a metering valve 201 for metering an amount of the fluid exiting the canister 202 corresponding to a single spray pattern or spray plume. The canister 202 may further include a valve stem 204 extending from the metering valve 201. The valve stem 204 may have a generally hollow structure. The canister 202 may be adapted to release a predetermined amount of the fluid through the metering valve 201 upon actuation. The fluid may be released through a delivery opening 114 of the actuator 100. A cover and a retaining member of the inhaler 200 is not shown in Figure 1 for the purpose of clarity.

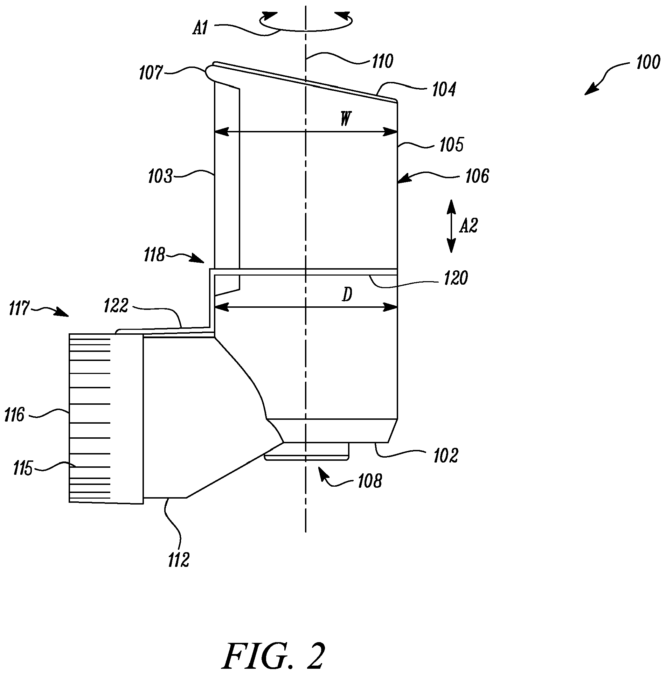

Figure 2 illustrates the actuator 100 of the inhaler 200 without the canister 202 inserted therein. Referring to Figures 1 and 2, the actuator 100 includes a sleeve 106 having a first end 102 and a second end 104. The sleeve 106 of the actuator 100 is substantially hollow. The first end 102 may be at a bottom of the actuator 100 and the sleeve 106. The second end 104 may be at a top of the actuator 100 and the sleeve 106. The sleeve 106 may therefore be a hollow tubular component. The canister 202 may be received within the sleeve 106 towards the second end 104 of the sleeve 106 such that at least a portion of the canister 202 extends outwards of the sleeve 106 from the second end 104. The sleeve 106 may also include a front end 103 and a rear end 105. The front end 103 may be at a left side of the actuator 100 and the sleeve 106 as shown in Figure 2. The rear end 105 may be at a right side of the actuator 100 and the sleeve 106 as also shown in Figure 2. Further, the sleeve 106 defines a longitudinal axis 110 along its length. In some embodiments, the longitudinal axis 110 may be substantially vertical.

The actuator 100 further includes a base 108 formed at the first end 102 of the sleeve 106, i.e., the base 108 is formed at the bottom of the actuator 100. Therefore, the hollow structure of the sleeve 106 may terminate at the first end 102 with the base 108.

The actuator 100 may include a nozzle block 208 (shown in Figure 1) formed at an inner surface of the base 108. The nozzle block 208 may have a substantially hollow tubular shape. The hollow tubular shape of the nozzle block 208 may help the nozzle block 208 to receive the valve stem 204 of the canister 202 within the nozzle block 208. The nozzle block 208 may extend upwardly from the base 108. The nozzle block 208 may include a seat 210 and an orifice 212. The orifice 212 may be fluidly coupled with the seat 210. The orifice 212 may be operable for dispensing the spray pattern or spray plume of metered fluid. Moreover, the base 108 may also define a grip section (not shown) at its outside surface. The grip section may aid a user to improve grip on the actuator 100 while using the inhaler 200. The grip section may essentially be a set of protrusions, a set of indents, or any other such structural arrangement.

The actuator 100 may be formed by any suitable material and process. For example, the actuator 100 may be formed with a polyolefin material (e.g., polyethylene or polypropylene), by processes such as injection moulding, compression moulding, rotational moulding, 3-D printing, and the like.

The actuator 100 further includes a mouthpiece 112 joined to the sleeve 106 and the base 108. The mouthpiece 112 may be integrally formed with the sleeve 106. The mouthpiece 112 is substantially hollow and defines a delivery opening 114 (shown in Figure 1). The inhaler 200 may be actuated to deliver the medicament from the canister 202 through the delivery opening 114. The delivery opening 114 is disposed at an end of the mouthpiece 112 distal to the sleeve 106. A front end 103 of the sleeve 106 is disposed proximal to the mouthpiece 112, while a rear end 105 of the sleeve 106 is disposed distal to the mouthpiece 112.

The actuator 100 further includes a cover 116. The mouthpiece 112 may be covered by the cover 116 to protect the delivery opening 114 from contaminants such as, fluff, lint, dust, etc. Specifically, the cover 116 is removably attached to the mouthpiece 112 for covering the delivery opening 114. The cover 116 may be detachably connected to the mouthpiece 112 by a snap-fit connection. During use of the inhaler 200, the cover 116 can be removed from the mouthpiece 112 to expose the delivery opening 114. The cover 116 may have any suitable shape based on a shape of the mouthpiece 112. The cover 116 may have protrusions or grooves 115 to facilitate gripping by a user.

The actuator 100 further includes a retaining member 118 for retaining the cover 116 to the sleeve 106 upon removal of the cover 116 from the mouthpiece 112. The retaining member 118 and the cover 116 may form a cover assembly 117. In other words, the cover assembly 117 includes the retaining member 118 and the cover 116.

The retaining member 118 includes a ring 120 disposed around the sleeve 106 and a strap 122 extending from the ring 120 and attached to the cover 116. The ring 120 is at least selectively rotatable (indicated by an arrow A1) about the longitudinal axis 110 and slidable (indicated by an arrow A2) along the longitudinal axis 110 relative to the sleeve 106. The ring 120 may have a generally annular shape. The ring 120 is both rotatable and slidable relative to the sleeve 106. The strap 122 may be deformable and may deform in a user desired position without much resistance. Accordingly, the retaining member 118 may be made of any suitable material which may allow deformation of the strap 122. It may be preferable to manufacture the components of the retaining member 118 integrally with each other. Therefore, the retaining member 118 may be preferably made of injection moulded polypropylene.

In some embodiments, a diameter D of the ring 120 is greater than a width W of the sleeve 106, i.e., the ring 120 may be disposed in a slight clearance fit with the sleeve 106. The diameter D corresponds to an inner diameter of the ring 120 that is disposed around the sleeve 106. In some embodiments, the diameter D of the ring 120 may be about 0.2 mm greater than the width W of the sleeve 106. In some embodiments, the diameter D of the ring 120 may be about 0.5 mm greater than the width W of the sleeve 106.

In some embodiments, the sleeve 106 of the actuator 100 further includes a protrusion 107 at the second end 104 of the sleeve 106. The protrusion 107 may aid to retain the ring 120 around the sleeve 106. The protrusion 107 may extend away from the longitudinal axis 110 of the sleeve 106. Further, the protrusion 107 may extend beyond the width W of the sleeve 106.

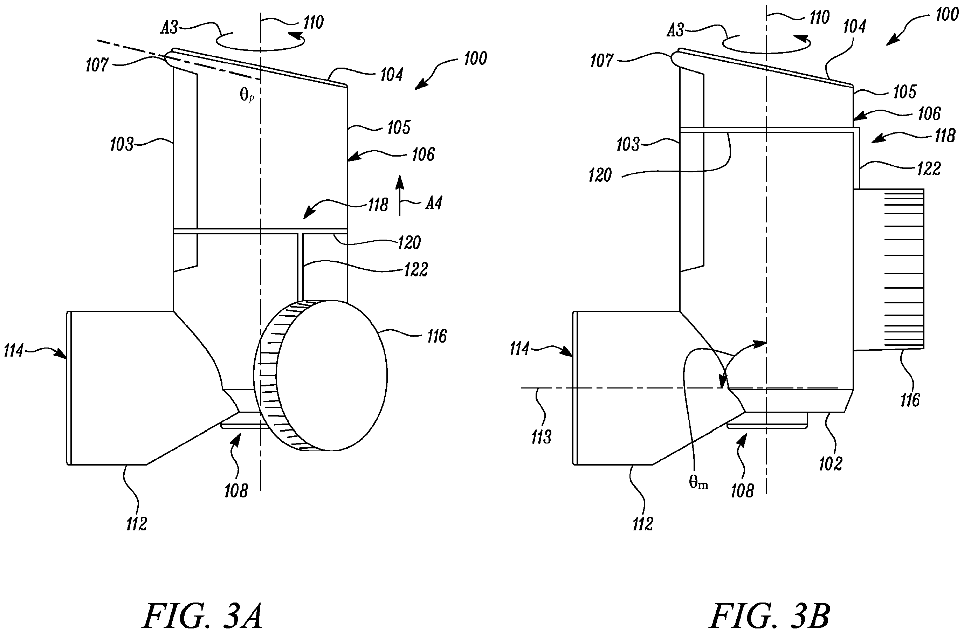

As shown in Figures 3A and 3B, the ring 120 may be both slidable and rotatable with respect to the sleeve 106 between the protrusion 107 and the mouthpiece 112. Accordingly, the protrusion 107 is inclined relative to the longitudinal axis 110 of the sleeve 106. The protrusion 107 may be inclined relative to the longitudinal axis 110 at an angle θₚ of about 90 degrees to about 120 degrees. In some embodiments, the protrusion 107 may be inclined relative to the longitudinal axis 110 at an angle θₚ of about 100 degrees to about 120 degrees. These are only exemplary values of the inclination angle θₚ of the protrusion 107 and any suitable angle might be chosen as per application requirements. By inclining the protrusion 107, it may be possible to assemble the ring 120 over the sleeve 106 as a 'snap fit', thereby making it difficult to remove the ring 120 again by accident. In other words, the protrusion 107 may prevent the ring 120 from being accidentally removed from the sleeve 106 at the second end 104.

It should be noted that the protrusion 107 is adapted to retain the ring 120 to the sleeve 106. In other words, the protrusion 107 may retain the ring 120 to the sleeve 106 when the ring 120 is slid towards the second end 104. The protrusion 107 may be added to current designs of inhalers. In some embodiments, the sleeve 106 of the actuator 100 may have increased width at the second end 104 such that the diameter D of the ring 120 is less than the width at the second end 104, nullifying the need to include the protrusion 107.

Figures 3A and 3B illustrate two of the many possible positions of the ring 120. As illustrated in Figure 3A, the cover 116 may be detached from the mouthpiece 112, thereby exposing the delivery opening 114. Further, the ring 120 is rotated about the longitudinal axis 110. For some users, this may be a suitable position for the cover 116 for using the inhaler 200.

Now referring to Figure 3B, the ring 120 is further rotated and slid along the longitudinal axis 110 relative to the sleeve 106. Referring to both Figures 3A and 3B, the rotation of the ring 120 is indicated by an arrow A3, while the sliding of the ring 120 is indicated by an arrow A4. The ring 120 is rotated and slid such that the cover 116 is moved away from the mouthpiece 112. The position of the ring 120 shown in Figure 3B is upwards of the position of the ring 120 in Figure 3A. In other words, the ring 120 is slid towards the second end 104 of the sleeve 106 such that the cover 116 is moved away from the mouthpiece 112. Further, the ring 120 is rotated such that the cover 116 is disposed proximal to the rear end 105 of the sleeve 106. For some users, this may be a suitable position for the cover 116 for using the inhaler 200. Such a position of the cover 116 may allow the inhaler 200 (shown in Figure 1) to be used with any interference from the cover 116. For example, the position of the cover 116 may facilitate handling of the inhaler 200 to manually dispense the medicament from the inhaler 200. The strap 122 may deform to allow various movements of the ring 120 relative to the sleeve 106.

Further, the mouthpiece 112 may define a mouthpiece axis 113 (shown in Figure 3B). The mouthpiece axis 113 of the mouthpiece 112 may define a mouthpiece angle θₘ relative to the longitudinal axis 110 of the sleeve 106. Therefore, the mouthpiece 112 is inclined relative to the longitudinal axis 110. In some embodiments, the mouthpiece 112 is substantially perpendicular to the longitudinal axis 110, i.e., the mouthpiece angle θₘ is about 90 degrees. In some embodiments, the mouthpiece angle θₘ may be from about 70 degrees to about 90 degrees. In some embodiments, the mouthpiece angle θₘ may be from about 90 degrees to about 110 degrees. These are only exemplary values of the mouthpiece angle θₘ and any suitable mouthpiece angle θₘ might be chosen as per application requirements.

In some embodiments, the ring 120 is substantially perpendicular to the longitudinal axis 110 (as shown in Figures 3A and 3B). The ring 120 is both rotatable and slidable relative to the sleeve 106. However, during use of the inhaler 200 (shown in Figure 1), the ring 120 may be selectively rotated about the longitudinal axis 110 or slid along the longitudinal axis 110 relative to the sleeve 106 or combinations thereof.

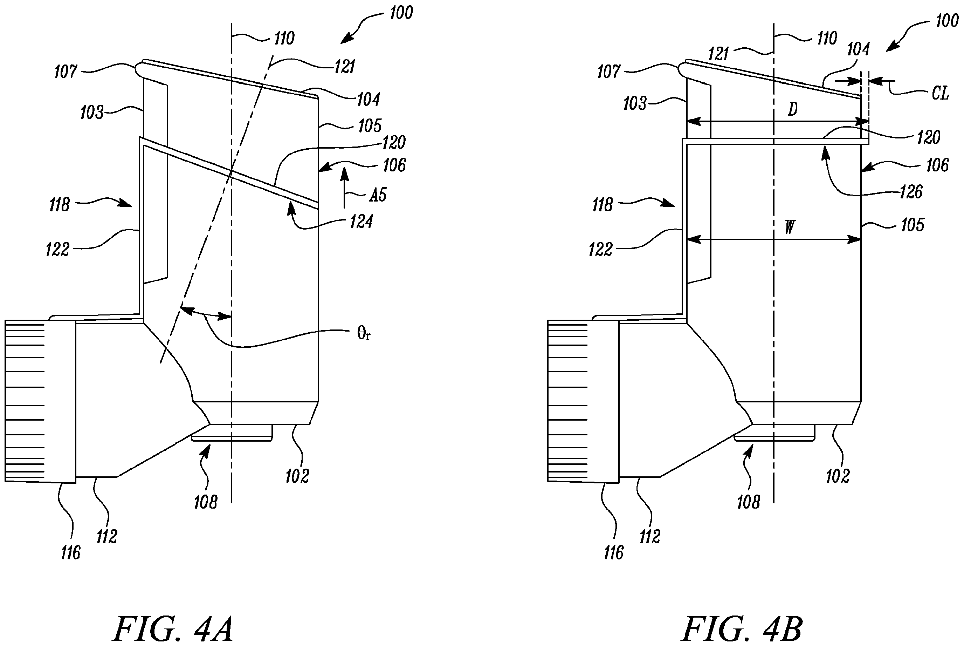

Referring to Figures 4A and 4B, another embodiment of the actuator 100 is shown. The ring 120 is movable between a locked position 124 and an unlocked position 126. The locked position 124 is shown in Figure 4A. The unlocked position 126 is shown in Figure 4B. Furthermore, in the locked position 124, the ring 120 is inclined towards the base 108. In other words, the ring 120 is upwards at the front end 103 of the sleeve 106 and downwards at the rear end 105 of the sleeve 106.

As illustrated in Figures 4A and B, the ring 120 may define a ring axis 121. The ring axis 121 may extend through a center of the ring 120. The ring axis 121 of the ring 120 may define a ring angle θᵣ relative to the longitudinal axis 110. In some embodiments, the ring axis 121 can be substantially parallel to the longitudinal axis 110, e.g., the ring angle θᵣ is about 0 degrees as shown in Figure 4B. In some embodiments, the ring angle θᵣ is no greater than about 10 degrees. In some embodiments, the ring angle θᵣ may be from about 10 degrees to about 30 degrees. In some embodiments, the ring angle θᵣ may be from about 20 degrees to about 30 degrees. These are only exemplary values of the ring angle θᵣ and any suitable ring angle θᵣ might be chosen as per application requirements.

The clearance fit of the ring 120 relative to the sleeve 106 may be greater than the embodiment illustrated in Figures 3A and 3B. For example, the diameter D of the ring 120 may be about 1 mm greater than the width W of the sleeve 106. In another example, the diameter D of the ring 120 may be about 1.5 mm greater than the width W of the sleeve 106. Furthermore, the ring angle θᵣ of the ring 120 may depend on a clearance between the diameter D of the ring 120 and the width W of the sleeve 106.

In the locked position 124, the ring 120 is inclined relative to the longitudinal axis 110 and locked with the sleeve 106 to prevent relative movement between the sleeve 106 and the ring 120. In the locked position 124, the ring 120 may be prevented from any substantial rotation or sliding relative to the sleeve 106. In some embodiments, for locking the ring 120 in the locked position 124, there may be a set of protrusions, a set of indents, or any other such structural arrangement at the front end 103 of the sleeve 106. In some embodiments, a coefficient of friction between the ring 120 and the sleeve 106 may be sufficient to keep the 120 in the locked position 124.

As illustrated in Figure 4B, in the unlocked position 126, the ring axis 121 is substantially parallel to the longitudinal axis 110 and is both rotatable and slidable relative to the sleeve 106. In the unlocked position 126, the ring 120 has a substantial clearance CL with respect to the sleeve 106. In order to bring the ring 120 into the unlocked position 126 from the locked position 124, the ring 120 may be lifted up (indicated by an arrow A5 in Figure 4A) from the rear end 105 of the sleeve 106 in such a manner that the ring axis 121 is substantially parallel to the longitudinal axis 110. In other words, in the unlocked position 126, the ring axis 121 (shown in Figure 4A) may be substantially parallel to the longitudinal axis 110.

Users of the inhaler 200 may decide a suitable position for the cover 116 during inhalation. For example, the suitable position for users may depend on their hand size, whether they are right-handed, left-handed or ambidextrous, whether they are a "thumb on top" pusher, a "fingers across the top, and thumb underneath" firer, a "hand wrapped round" holder, and/or whether they are a carer administering a dose to another person. The cover 116 may be positioned to suit them without the cover 116 becoming lost. Two of the many possible suitable positions are illustrated in Figures 3A and 3B.

In an aspect, the inhaler 200 is provided. The inhaler 200 is shown in Figure 1. The inhaler 200 may be formed by injection moulding of a polyolefin material (e.g., polyethylene or polypropylene). The inhaler 200 includes the aforementioned actuator 100. The inhaler 200 further includes the canister 202. The canister 202 contains a fluid formulated with a medicament. The canister 202 is at least partially received within the sleeve 106 of the actuator 100.

In another aspect, a method of using the inhaler 200 of the present disclosure includes the following steps. The method is explained with reference to Figures 1, 2, 3A and 3B. The first step includes detaching the cover 116 from the mouthpiece 112 to at least partially expose the delivery opening 114. During this step, the cover 116 is detached from the mouthpiece 112.

The second step includes rotating the ring 120 relative to the sleeve 106 to move the cover 116 away from the mouthpiece 112. In some cases, the second step further incudes sliding the ring 120 towards the second end 104 of the sleeve 106. The ring 120 may be rotated to any user desired position.

The third step includes actuating the inhaler 200 to deliver the medicament through the delivery opening. Actuating the inhaler 200 may include pressing the canister 202 against the base 108 of the sleeve 106 of the actuator 100.

In some aspects, a method of using the inhaler 200 of the present disclosure includes the following steps. The method is explained with reference to Figures 1, 2, 3A, 3B, 4A and 4B.

The first step includes detaching the cover 116 from the mouthpiece 112 to at least partially expose the delivery opening 114. During this step, the cover 116 is detached from the mouthpiece 112.

The second step includes lifting the ring 120 from the rear end 105 of the sleeve 106 of the actuator 100 in a manner that the ring axis 121 is substantially parallel to the longitudinal axis 110. In other words, the ring 120 is brought to the unlocked position 126 from the locked position 124.

The third step includes rotating the ring 120 relative to the sleeve 106 to move the cover 116 away from the mouthpiece 112. In some cases, the third step further incudes sliding the ring 120 towards the second end 104 of the sleeve 106. The ring 120 may be rotated to any user desired position.

The fourth step includes actuating the inhaler 200 to deliver the medicament through the delivery opening. Actuating the inhaler 200 may include pressing the canister 202 against the base 108 of the sleeve 106 of the actuator 100.

It will be known to one skilled in the art that numerous embodiments of the disclosure are possible. For example, the ring 120 may not be completely continuous and may be in segments. In another example, there may be multiple straps 122 attached to the cover 116. The disclosure is also equally applicable to Dry Powder Inhalers (DPIs), soft mist inhalers, etc.

Unless otherwise indicated, all numbers expressing feature sizes, amounts, and physical properties used in the specification and claims are to be understood as being modified by the term "about". Accordingly, unless indicated to the contrary, the numerical parameters set forth in the foregoing specification and attached claims are approximations that can vary depending upon the desired properties sought to be obtained by those skilled in the art utilizing the teachings disclosed herein.

Although specific embodiments have been illustrated and described herein, it will be appreciated by those of ordinary skill in the art that a variety of alternate and/or equivalent implementations can be substituted for the specific embodiments shown and described without departing from the scope of the present disclosure. This application is intended to cover any adaptations or variations of the specific embodiments discussed herein. Therefore, it is intended that the invention be limited only by the claims which follow.

## Claims

1. An actuator (100) for an inhaler (200), the actuator (100) comprising:
a sleeve (106) having a first end (102) and a second end (104), wherein the sleeve (106) is substantially hollow and defines a longitudinal axis (110) along its length;
a base (108) formed at the first end (102) of the sleeve (106);
a mouthpiece (112) joined to the sleeve (106) and the base (108), wherein the mouthpiece (112) is substantially hollow and defines a delivery opening (114); and
a cover (116) removably attached to the mouthpiece (112) for covering the delivery opening (114);
**characterized in that** the actuator (100) comprises:
a retaining member (118) for retaining the cover (116) to the sleeve (106) upon removal of the cover (116) from the mouthpiece (112), the retaining member (118) comprising:
a ring (120) disposed around the sleeve (106), wherein the ring (120) is at least selectively rotatable about the longitudinal axis (110) and slidable along the longitudinal axis (110) relative to the sleeve (106); and
a strap (122) extending from the ring (120) and attached to the cover (116).

2. The actuator (100) according to claim 1, wherein the ring (120) defines a ring axis (121) that extends through a center of the ring, wherein the ring axis (121) is substantially parallel to the longitudinal axis (110), and further wherein the ring (120) is both rotatable and slidable relative to the sleeve (106).

3. The actuator (100) according to claim 1, wherein the ring (120) is movable between a locked position (124) and an unlocked position (126), wherein:
in the locked position (124), the ring (120) is inclined relative to the longitudinal axis (110) and locked with the sleeve (106) to prevent relative movement between the sleeve (106) and the ring (120); and
in the unlocked position (126), the ring (120) is substantially parallel to the longitudinal axis (110) and is both rotatable and slidable relative to the sleeve (106).

4. The actuator (100) according to claim 3, wherein, in the locked position (124), the ring (120) is inclined towards the base (108).

5. The actuator (100) according to claim 1, wherein the mouthpiece (112) is inclined relative to the longitudinal axis (110).

6. The actuator (100) according to claim 5, wherein the mouthpiece (112) is substantially perpendicular to the longitudinal axis (110).

7. The actuator (100) according to claim 1, wherein the sleeve (106) further comprises a protrusion (107) at the second end (104) thereof, and wherein the protrusion (107) is inclined relative to the longitudinal axis (110).

8. The actuator (100) according to claim 1, wherein a diameter (D) of the ring (120) is greater than a width (W) of the sleeve (106).

9. An inhaler (200) comprising:
the actuator (100) of claim 1; and
a canister (202) containing a fluid formulated with a medicament, wherein the canister (202) is at least partially received within the sleeve (106) of the actuator (100).

10. The inhaler (200) according to claim 9, wherein the ring (120) defines a ring axis (121) that extends through a center of the ring (120), wherein the ring axis (121) is substantially parallel to the longitudinal axis (110), and further wherein the ring (120) is both rotatable and slidable relative to the sleeve (106).

11. The inhaler (200) according to claim 9, wherein the ring (120) is movable between a locked position (124) and an unlocked position (126), wherein:
in the locked position (124), the ring (120) is inclined relative to the longitudinal axis (110) and locked with the sleeve (106) to prevent relative movement between the sleeve (106) and the ring (120); and
in the unlocked position (126), a ring axis (121) defined by the ring (120) and that extends through a center of the ring (120) is substantially parallel to the longitudinal axis (110) and is both rotatable and slidable relative to the sleeve (106).

12. The inhaler (200) according to claim 11, wherein, in the locked position (124), the ring (120) is inclined towards the base.

13. The inhaler (200) according to claim 9, wherein the mouthpiece (112) is inclined relative to the longitudinal axis (110).

14. The inhaler (200) according to claim 13, wherein the mouthpiece (112) is substantially perpendicular to the longitudinal axis (110).

15. The inhaler (200) according to claim 9, wherein the sleeve (106) further comprises a protrusion (107) at the second end (104) thereof, and wherein the protrusion (107) is inclined relative to the longitudinal axis (110).

## Patentansprüche

1. Aktuator (100) für einen Inhalator (200), wobei der Aktuator (100) aufweist:
eine Hülse (106) mit einem ersten Ende (102) und einem zweiten Ende (104), wobei die Hülse (106) im Wesentlichen hohl ist und eine Längsachse (110) entlang ihrer Länge definiert,
eine am ersten Ende (102) der Hülse (106) ausgebildete Basis (108);
ein mit der Hülse (106) und der Basis (108) verbundenes Mundstück (112), wobei das Mundstück (112) im Wesentlichen hohl ist und eine Abgabeöffnung (114) definiert, und
eine abnehmbar am Mundstück (112) befestigte Abdeckung (116) zum Abdecken der Abgabeöffnung (114);
**dadurch gekennzeichnet, dass** der Aktuator (100) aufweist:
ein Halteelement (118) zum Halten der Abdeckung (116) an der Hülse (106) beim Entfernen der Abdeckung (116) vom Mundstück (112), wobei das Halteelement (118) aufweist:
einen um die Hülse (106) herum angeordneten Ring (120), wobei der Ring (120) zumindest selektiv um die Längsachse (110) drehbar und entlang der Längsachse (110) relativ zur Hülse (106) verschiebbar ist; und
einen Streifen (122), der sich vom Ring (120) erstreckt und an der Abdeckung (116) befestigt ist.

2. Aktuator (100) nach Anspruch 1, wobei der Ring (120) eine sich durch eine Mitte des Rings erstreckende Ringachse (121) definiert, wobei die Ringachse (121) sich im Wesentlichen parallel zur Längsachse (110) erstreckt, und wobei ferner der Ring (120) relativ zur Hülse (106) sowohl drehbar als auch verschiebbar ist.

3. Aktuator (100) nach Anspruch 1, wobei der Ring (120) zwischen einer verriegelten Position (124) und einer entriegelten Position (126) bewegbar ist, wobei:
der Ring (120) in der verriegelten Position (124) relativ zur Längsachse (110) geneigt und mit der Hülse (106) verriegelt ist, um eine Relativbewegung zwischen der Hülse (106) und dem Ring (120) zu verhindern, und
der Ring (120) in der entriegelten Position (126) sich im Wesentlichen parallel zur Längsachse (110) erstreckt und relativ zur Hülse (106) sowohl drehbar als auch verschiebbar ist.

4. Aktuator (100) nach Anspruch 3, wobei der Ring (120) in der verriegelten Position (124) zur Basis (108) hin geneigt ist.

5. Aktuator (100) nach Anspruch 1, wobei das Mundstück (112) relativ zur Längsachse (110) geneigt ist.

6. Aktuator (100) nach Anspruch 5, wobei das Mundstück (112) sich im Wesentlichen senkrecht zur Längsachse (110) erstreckt.

7. Aktuator (100) nach Anspruch 1, wobei die Hülse (106) ferner einen Vorsprung (107) an ihrem zweiten Ende (104) aufweist, und wobei der Vorsprung (107) relativ zur Längsachse (110) geneigt ist.

8. Aktuator (100) nach Anspruch 1, wobei ein Durchmesser (D) des Rings (120) größer ist als eine Breite (W) der Hülse (106).

9. Inhalator (200), mit:
dem Aktuator (100) nach Anspruch 1; und
einem Behälter (202), der ein mit einem Medikament formuliertes Fluid enthält, wobei der Behälter (202) zumindest teilweise innerhalb der Hülse (106) des Aktuators (100) aufgenommen ist.

10. Inhalator (200) nach Anspruch 9, wobei der Ring (120) eine Ringachse (121) definiert, die sich durch eine Mitte des Rings (120) erstreckt, wobei die Ringachse (121) sich im Wesentlichen parallel zur Längsachse (110) erstreckt, und wobei ferner der Ring (120) relativ zur Hülse (106) sowohl drehbar als auch verschiebbar ist.

11. Inhalator (200) nach Anspruch 9, wobei der Ring (120) zwischen einer verriegelten Position (124) und einer entriegelten Position (126) bewegbar ist, wobei:
der Ring (120) in der verriegelten Position (124) relativ zur Längsachse (110) geneigt und mit der Hülse (106) verriegelt ist, um eine Relativbewegung zwischen der Hülse (106) und dem Ring (120) zu verhindern, und
in der entriegelten Position (126) eine Ringachse (121), die durch den Ring (120) definiert ist und sich durch eine Mitte des Rings (120) erstreckt, sich im Wesentlichen parallel zur Längsachse (110) erstreckt und relativ zur Hülse (106) sowohl drehbar als auch verschiebbar ist.

12. Inhalator (200) nach Anspruch 11, wobei der Ring (120) in der verriegelten Position (124) zur Basis hin geneigt ist.

13. Inhalator (200) nach Anspruch 9, wobei das Mundstück (112) relativ zur Längsachse (110) geneigt ist.

14. Inhalator (200) nach Anspruch 13, wobei das Mundstück (112) sich im Wesentlichen senkrecht zur Längsachse (110) erstreckt.

15. Inhalator (200) nach Anspruch 9, wobei die Hülse (106) ferner einen Vorsprung (107) an ihrem zweiten Ende (104) aufweist, und wobei der Vorsprung (107) relativ zur Längsachse (10) geneigt ist.

## Revendications

1. Actionneur (100) pour un inhalateur (200), l'actionneur (100) comprenant :
un manchon (106) ayant une première extrémité (102) et une deuxième extrémité (104), dans lequel le manchon (106) est sensiblement creux et définit un axe longitudinal (110) le long de sa longueur ;
une base (108) formée au niveau de la première extrémité (102) du manchon (106) ;
un embout buccal (112) assemblé au manchon (106) et à la base (108), dans lequel l'embout buccal (112) est sensiblement creux et définit une ouverture de distribution (114) ; et
un couvercle (116) fixé, de manière amovible, à l'embout buccal (112) pour recouvrir l'ouverture de distribution (114) ;
**caractérisé en ce que** l'actionneur (100) comprend :
un élément de retenue (118) pour retenir le couvercle (116) sur le manchon (106) suite au retrait du couvercle (116) de l'embout buccal (112), l'élément de retenue (118) comprenant :
une bague (120) disposée autour du manchon (106), dans lequel la bague (120) peut au moins sélectivement tourner autour de l'axe longitudinal (110) et coulisser le long de l'axe longitudinal (110) par rapport au manchon (106) ; et
une sangle (122) s'étendant à partir de la bague (120) et fixée sur le couvercle (116).

2. Actionneur (100) selon la revendication 1, dans lequel la bague (120) définit un axe de bague (121) qui s'étend à travers un centre de la bague, dans lequel l'axe de bague (121) est sensiblement parallèle à l'axe longitudinal (110), et en outre dans lequel la bague (120) peut à la fois tourner et coulisser par rapport au manchon (106).

3. Actionneur (100) selon la revendication 1, dans lequel la bague (120) est mobile entre une position verrouillée (124) et une position déverrouillée (126), dans lequel :
dans la position verrouillée (124), la bague (120) est inclinée par rapport à l'axe longitudinal (110) et verrouillée avec le manchon (106) pour empêcher le mouvement relatif entre le manchon (106) et la bague (120) ; et
dans la position déverrouillée (126), la bague (120) est sensiblement parallèle à l'axe longitudinal (110) et peut à la fois tourner et coulisser par rapport au manchon (106).

4. Actionneur (100) selon la revendication 3, dans lequel, dans la position verrouillée (124), la bague (120) est inclinée vers la base (108).

5. Actionneur (100) selon la revendication 1, dans lequel l'embout buccal (112) est incliné par rapport à l'axe longitudinal (110).

6. Actionneur (100) selon la revendication 5, dans lequel l'embout buccal (112) est sensiblement perpendiculaire à l'axe longitudinal (110).

7. Actionneur (100) selon la revendication 1, dans lequel le manchon (106) comprend en outre une saillie (107) au niveau de sa deuxième extrémité (104), et dans lequel la saillie (107) est inclinée par rapport à l'axe longitudinal (110).

8. Actionneur (100) selon la revendication 1, dans lequel un diamètre (D) de la bague (120) est supérieur à une largeur (W) du manchon (106).

9. Inhalateur (200) comprenant :
l'actionneur (100) selon la revendication 1 ; et
une cartouche (202) contenant un fluide formulé avec un médicament, dans lequel la cartouche (202) est au moins partiellement reçue à l'intérieur du manchon (106) de l'actionneur (100).

10. Inhalateur (200) selon la revendication 9, dans lequel la bague (120) définit un axe de bague (121) qui s'étend à travers un centre de la bague (120), dans lequel l'axe de bague (121) est sensiblement parallèle à l'axe longitudinal (110), et en outre dans lequel la bague (120) peut à la fois tourner et coulisser par rapport au manchon (106).

11. Inhalateur (200) selon la revendication 9, dans lequel la bague (120) est mobile entre une position verrouillée (124) et une position déverrouillée (126), dans lequel :
dans la position verrouillée (124), la bague (120) est inclinée par rapport à l'axe longitudinal (110) et verrouillée avec le manchon (106) pour empêcher le mouvement relatif entre le manchon (106) et la bague (120) ; et
dans la position déverrouillée (126), un axe de bague (121) défini par la bague (120) et qui s'étend à travers un centre de la bague (120), est sensiblement parallèle à l'axe longitudinal (110) et peut à la fois tourner et coulisser par rapport au manchon (106).

12. Inhalateur (200) selon la revendication 11, dans lequel, dans la position verrouillée (124), la bague (120) est inclinée vers la base.

13. Inhalateur (200) selon la revendication 9, dans lequel l'embout buccal (112) est incliné par rapport à l'axe longitudinal (110).

14. Inhalateur (200) selon la revendication 13, dans lequel l'embout buccal (112) est sensiblement perpendiculaire à l'axe longitudinal (110).

15. Inhalateur (200) selon la revendication 9, dans lequel le manchon (106) comprend en outre une saillie (107) au niveau de sa deuxième extrémité (104), et dans lequel la saillie (107) est inclinée par rapport à l'axe longitudinal (110).
